# EUROPEAN PATENT SPECIFICATION

(11) **EP 1 065 985 B1**
(45) Date of publication and mention of the grant of the patent: **02.08.2006**
(21) Application number: 00910042.1
(22) Date of filing: 01.02.2000
(51) Int. Cl.: A61B 18/14

(54) **IMPROVED RF BIPOLAR END EFFECTOR FOR USE IN ELECTROSURGICAL INSTRUMENTS**
VERBESSERTES, MIT RADIOFREQUENZ ARBEITENDES, BIPOLARES ENDSTÜCK ZUR ANWENDUNG IN ELEKTROCHIRURGISCHEN INSTRUMENTEN
EFFECTEUR BIPOLAIRE RF PERFECTIONNE, UTILE DANS DES INSTRUMENTS ELECTROCHIRURGICAUX

(30) Priority: 02.02.1999 US 241575
(43) Date of publication of application: 10.01.2001
(73) Proprietor: ETHICON ENDO-SURGERY, INC., Cincinnati, OH 45242-2839 (US)
(72) Inventor: WAMPLER, Scott, D., West Chester, OH 45069 (US); COLLINS, William, L., Jr., Cincinnati, OH 45241 (US); YATES, David, C., West Chester, OH 45069 (US)
(74) Representative: Mercer, Christopher Paul
(86) International application number: PCT/US2000/002562
(87) International publication number: WO 2000/045726

(56) References cited:
- US-A- 5 693 051
- US-A- 5 833 690

## Description

This application is related to the following co-pending patent applications: U.S. Patent application Serial No. 60/073,890 [Attorney Docket No. END-514].

### Field of the Invention

The present invention relates, in general, to an improved bipolar electrode configuration for use in bipolar electrosurgical instruments and, more particularly, to a bipolar electrode configuration including electrodes arranged around a fixed height tissue slot.

### Background of the Invention

RF (Radio Frequency) electricity has been used for decades to cauterize and coagulate tissue in surgical procedures. Devices used to apply RF energy to tissue fall generally into two categories: mono-polar and bipolar. Bipolar electrosurgical instruments incorporate both active and return electrodes into the surgical instrument, substantially restricting the flow of electric current to tissue that is placed between the electrodes. In mono-polar electrosurgical instruments, on the other hand, the return electrode is placed outside the patients body, on the patients skin. Thus, in a mono-polar electrosurgical instrument, current flows from the active or treatment electrode through the patients body to the return electrode. Both mono-polar and bipolar electrosurgical instruments rely, at least in part, upon resistance healing to treat (e.g. cauterize and/or cut) tissue. As current is passed through tissue, the electrical resistance of the tissue results in the dissipation of power in the form of heat. As the temperature of the tissue rises, its characteristics, including electrical resistance, change. When the tissue temperature reaches approximately 67-70 degrees C, coagulation begins. As additional energy is dissipated in the tissue collagen, which forms the backbone of the tissue matrix, continues to break down and "melt". Once the collagen begins to break down, the tissue begins to coagulate. When the collagen begins to break down, compression of the tissue will cause the compressed tissue layers to fuse, sealing adjacent blood vessels- When the tissue temperature reaches one hundred degrees C most fluids (deluding water) are driven off or evaporated from the tissue, desiccating the tissue and substantially increasing the electrical resistance of the tissue. The desiccated tissue may then be cut or separated with little effort The rate at which energy is dissipated into tissue is dependent on many factors, including the electrical resistance of the tissue and the density of the electric current flowing through the tissue. Since electrosurgical instruments are generally designed to be used to treat a variety of tissue types, current density becomes an important design consideration, and, particularly in bipolar electrosurgical devices, current density is, for a particular tissue type, a function of the number, size, shape and placement of the device electrodes.

In many surgical applications, it is desirable to use bipolar electrical energy as a means of cutting and/or coagulating tissue. In bipolar electrosurgical instruments, it is generally desirable to ensure that the flow of electric current is confined to the tissue in the instrument and, to a significantly lesser extent to the tissue adjacent the instrument. Generally, in prior art bipolar electrosurgical instruments, these goals have been accomplished by designing an instrument which grasps or clamps the tissue prior to the application of electrosurgical energy. See for example US 5833690. Such bipolar electrosurgical instruments are well known in the art and, in particular, many designs have been suggested for surgical instruments which coagulate tissue either prior to cutting the tissue or during the cutting process. In most of these instruments, the tissue is first grasped by jaws which apply pressure to the tissue prior to the application of electrosurgical energy. In such instruments, the grasping jaws either constitute or include the electrodes which supply the electrosurgical energy, although, in some designs, one or more of the electrodes may be incorporated into other elements of the instrument, including, for example, the cutting element. Thus, in such bipolar electrosurgical grasping instruments, the tissue being treated is first grasped, then electrosurgical energy is applied by the electrodes, then the tissue is cut or separated, and, finally, the tissue is released and the grasping instrument is moved to fresh tissue so that the process can be repeated. While this procedure is very effective in many surgical procedures, when working in certain types of tissue, such as mesentery tissue, it may become tedious to continuously grasp and release as the instrument is moved through the tissue. However, since tissue such as mesentery tissue is vascular and will bleed if the blood vessels are not adequately sealed, it is important to ensure that the blood in the tissue on either side of the cut line is thoroughly coagulated prior to separating the tissue. Further, since many modem surgical procedures are performed in very small spaces, there may not be sufficient room to use an instrument with jaws which must be opened after each application of electrosurgical energy.

It would, therefore, be advantageous to design a bipolar electrosurgical end effector adapted to coagulate and cut tissue while moving continuously through the tissue. It would further be advantageous to design a bipolar electrosurgical end effector adapted to coagulate and cut tissue, including vascular structures, while moving the end effector continuously through the tissue, wherein the coagulation region is substantially confined to the width of the jaw assembly. It would further be advantageous to design a bipolar electrosurgical end effector adapted to continuously receive, coagulate and divide the coagulated tissue as the electrosurgical end effector is moved through the tissue. It would further be advantageous to design a bipolar electrosurgical end effector adapted to continuously receive, coagulate and divide tissue wherein the electrosurgical current through the tissue in substantially self limiting.

### Summary of the Invention

The present invention is directed to a bipolar electrosurgical end effector for use in medical instruments as defined in the appended set of claims. A bipolar end effector according to the present invention includes: a first tissue surface; a first elongated electrode on a first side of the first tissue surface; and a second elongated electrode on a second side of the first tissue surface. The second electrode is generally substantially parallel to the first electrode. A bipolar end effector according to the present invention further includes: a first central insulation region separating the first electrode from the second electrode; a tissue slot separating a second tissue surface from the first and second electrodes; and a tissue separator positioned between the first and second tissue surfaces. The tissue separator generally includes: a dividing edge at a distal end of the tissue separator; a first tissue guide extending proximally away from the dividing edge toward the first side; and a second tissue guide extending proximally away from the dividing edge toward the second side.

In a further embodiment of the present invention, a bipolar electrosurgical end effector may include: a first elongated electrode on a first side of the end effector; a second elongated electrode on the first side of the end effector, wherein the second electrode is substantially parallel to the first electrode; a third elongated electrode on a second side of the end effector, wherein the third electrode is substantially parallel to the first and second electrodes; and a fourth elongated electrode on the second side of the end effector, wherein the fourth elongated electrode is substantially parallel to the first, second and third electrodes. In this embodiment of the invention, a first central insulation region separates the first electrode from the third electrode and a second central insulation region separates the second electrode from the fourth electrode, and a tissue separator is positioned between the first and the second central insulation regions proximal to the distal end of the end effector. The tissue separator may include: a dividing edge at the distal end of the tissue separator; a first tissue surface extending proximally away from the dividing edge toward the first side of the end effector; and a second tissue surface extending proximally away from the dividing edge toward the second side of the end effector.

Further embodiments of the present invention may include a bipolar electrosurgical end effector wherein the tissue separator comprises a wedge shaped region and a bipolar electrosurgical end effector wherein the first electrode is positioned substantially directly opposite the third electrode and the second electrode is positioned substantially directly opposite the fourth electrode. In addition, embodiments of the present invention may include a bipolar electrosurgical end effector wherein the first and second electrodes are electrically connected and the second and third electrodes are electrically connected.

### Specific Description

The novel features of the invention are set forth with particularity in the appended claims. The invention itself, however, both as to organization and methods of operation, together with further objects and advantages thereof, may best be understood by reference to the following description.

### Detailed Description of the Invention

A bipolar electrosurgical instrument according to the present invention includes a handle an extender tube, a jaw assembly and a power cord. The bipolar electrosurgical instrument also includes an electrosurgical generator and a foot switch. The Electrosurgical generator may be connected to an ac wall plug through a generator power cord. The generator may be, for example, an RF electrosurgery generator such as the generator available from ERBE Elektromedizin GmbH as model ICC 350. The electrosurgical generator may be controlled and actuated by a conventional foot switch which may be connected to the generator by a foot switch cord The electrosurgical generator may be connected to the bipolar electrosmgical instrument by a power cord which is adapted to carry bipolar electrosurgical energy to the bipolar electrosurgical instrument.

The end effector of the bipolar electrosurgical instrument includes a first electrode assembly a second electrode assembly , a central insulator assembly, a tissue separator and a tissue slot. The tissue separator may preferably comprise a central wedge which includes a leading edge a first wedge wall and a second wedge wall. While embodiments of tissue separators according to the present invention may include leading edges which are surgically sharp, that is sharp enough to cut through healthy tissue without tearing the tissue, it may, in certain circumstances, be preferable to utilize a tissue separator wherein the leading edge is surgically dull, that is too dull to readily cut healthy tissue but sharp enough to cut tissue which has been electrosurgically treated (e.g. desiccated tissue). The end effector includes a first electric wire and a second electric wire. The first electrical wire and second electrical wire are electrically connected to the power cord and adapted to transmit electrosurgical energy from the electrosurgical generator to the end effector the first electric wire includes a first electrical conductor and the second electrical wire includes a second electrical conductor. The first electrical conductor is connected to the first electrode assembly by, for example, silver soldering the first electrical conductor to the first electrode assembly the second electrical conductor is connected to the second electrode assembly by, for example, silver soldering the second electrical conductor to the second electrode assembly . The first electrode assembly includes a first electrode and a second electrode while the second electrode assembly includes a third electrode and a fourth electrode.

The first electrode assembly includes the first electrode and the second electrode which are electrically and mechanically connected by a first support member. Further, the second electrode assembly includes the third electrode and the fourth electrode which are electrically and mechanically connected by a second support member . In this embodiment of the invention, the first support member includes at least a portion of first wedge wall and the second support member includes at least a portion of a second wedge wall. The first electrode assembly also includes a first conductor notch and a second conductor notch which are adapted to receive the first electrical conductor and the second electrical conductor respectively. The first electrical conductor is electrically and mechanically connected to the first electrode assembly by, for example, soldering the first electrical conductor into the first conductor notch. The second electrical conductor is electrically and mechanically connected to the second electrode assembly by, for example, soldering the second electrical conductor into the second conductor notch. The central insulator assembly, which may be formed of an electrically non-conductive material in substantially the same shape as the first electrode assembly and the second electrode assembly electrically isolates the first electrode assembly from the second electrode assembly The central electrode assembly further includes a portion of the first wedge wall, the second wedge wall and the leading edge, which are made of an electrically non-conductive material.

The first tissue surface 52 includes a (first) contact surface of the first electrode and a (third) contact surface of the third electrode. The first tissue surface may further include a portion of the first central insulator which separates the first contact surface from the third contact surface. The tissue slot is bounded by the first tissue surface and the second tissue surface. The second tissue surface includes a (second) contact surface of second electrode and a (fourth) contact surface of the fourth electrode. The second tissue surface may further include a portion of a second central insulator which separates the second contact surface from the fourth contact surface, electrically isolating the second contact surface from the fourth contact surface. The tissue separator, including the leading edge the first wedge wall and the second wedge wall forms at least a portion of the proximal end of the tissue slot An outer insulating shell surrounds and isolates the elements of the end effector , supporting the end effector and electrically isolating the end effector from tissue outside of the tissue slot. The first and second tissue surfaces may also include at least a portion of the insulating shell. The insulating shell also includes an upper dissector and a lower dissector which each comprise partial cone shaped regions on either side of the distal end of the tissue slot. The upper dissector includes an upper ramped surface and the lower dissector includes a lower ramped surface which extend from the distal end of the jaw assembly to the distal opening of the tissue slot.

The tissue slot 50 is clearly visible with the upper ramped surface and the lower ramped surface leading into the tissue slot. The first electrode wire and the first electrical conductor extend from the jaw assembly throng the extender tube and the handle to the power cord.

The end effector is embedded in the insulating shell with the first contact surface and the second contact surface forming at least a portion of first tissue surface 4 and second tissue surface while the first electrical wire extends through the insulating shell and into the extender tube.

The tissue separator 40 includes the leading edge the first wall and the second wall. The first wall includes a portion of the central insulator assembly the first support member of the first electrode assembly and a portion of the insulating shell while the second wall includes a portion of the central insulator assembly a portion of the second support member of the second electrode assembly and a portion of the insulating shell.

The third and fourth electrodes are electrically connected through the support member while the first and second electrodes are electrically connected through and/or support member. The third and fourth electrodes are connected to a second electric wire which is connected to a first output electrosurgical generator by the bipolar power cord. The first and second electrodes are connected to a second electric wire which is connected to a second out put of the electrosurgical generator by the bipolar power cord. The electrodes are constructed of an electrically conductive material while the central insulators are constructed of material which is not electrically conductive. In the present embodiment of the invention, the working portion (i.e. the interior contact surfaces) of the end effector electrodes may be designed to be substantially C-shaped. After the wires are soldered to the electrode assemblies the electrode assemblies are stacked with the insulator assembly to form the end effector. A wedge shaped tissue separator may then be beveled into the backside of the tissue slot with the leading edge located at the center of the insulator assembly. Although the tissue separator is illustrated and described as being wedge shaped, a number of alternative shapes would be suitable for forming the tissue separator, including a long, thin section positioned substantially in the center of the tissue slot. Other suitable shapes which act to separate the tissue into two parts may be apparent to those skilled in the art.

In the embodiment of the invention described herein, there are a number of dimensions which may be adjusted to optimize the operation of the instrument for particular tissue types and particular surgical applications. In particular, W_{G} is the height of the tissue slot, Wₑ is the width of the electrode contact surface and Wᵢ is the width of the portion of the central insulator assembly which separates the electrode contact surfaces. More particularly, Wᵢ is the nominal distance between the first and third electrode contact surfaces and the second and fourth electrode contact surfaces along the length of the tissue slot. It is important that Wᵢ be wide enough to preclude dielectric break down between the electrodes. Further, the speed at which the tissue is desiccated may be controlled by the width Wᵢ; the larger Wᵢ is, the slower the instrument desiccates tissue and the smaller Wᵢ is, the faster the instrument desiccates tissue down to an optimal minimum value of approximately 0.5mm (.020") Wᵢ is preferably substantially constant along the length of the tissue slot since the first since electrode is preferably parallel to the third electrode along the length of the tissue slot and the second electrode is preferably parallel to the fourth electrode along the length of the tissue slot. Slot height W_{G} is preferably constant along the entire length of the tissue slot since the first and third contact surfaces are preferably parallel to the second and fourth contact surfaces respectively, along the entire length of the tissue slot Although a constant W_{G} is the preferable arrangement, it may be desirable in certain circumstances to design a tissue slot wherein the width of the tissue slot changes along its length, for example, it may be desirable to design a tissue slot which narrows from its distal opening to the tissue separator at its lateral end in order to increase the pressure on the treated tissue as it is moved through the tissue slot. However, in a bipolar electrosurgical instrument according to the present invention, the instrument is designed such that the height W_{G} at each point along the length of the tissue slot is generally fixed and unchanging during the time tissue is being moved through the tissue slot and treated. In a typical instrument according to the present invention, a preferred value for W_{G} would be in the range of approximately 0.5 mm (.020") 0.75 m (0.030") which would be expected to provide optimal compression of blood vessels ranging in size from approximately one to four millimeters in diameter as the blood vessels are moved into and along the tissue slot An instrument with a larger height W_{G} will be able to accept and treat a larger range of vessels (e.g. 4-7 mm) or thicker tissue. Such blood vessels may be found in, for example, side branches of the saphenous vein which must be sealed when the saphenous vein is harvested for use in, for example, arterial bypass operations. Once tissue, such as, for example, saphenous vein side branches are inserted into the tissue slot electrosurgical energy may be applied to the vein side blanches through the electrodes. It would further be expected, for reasons discussed below, that optimal cauterization of the tissue in the tissue slot would occur when W_{G} is less than twice of Wₑ. Longer tissue slot lengths may also be desirable since the more tissue engaged by the slot, the lower the total impedance and the faster the tissue cooks.

The bipolar electrosurgicla instrument according to the present invention may be used to cauterize and cut mesentery tissue including blood vessels in the mesentery tissue, during abdominal surgery.

In operation, tissue, such as mesentery tissue is inserted by a surgeon into the distal end of the tissue slot. Tissue is guided into the tissue slot by the upper ramped surface and the lower ramped surface while the upper dissector and the lower dissector serve to guide surrounding tissue away from the tissue slot, dissecting the tissue being treated from the surrounding tissue and preventing the surrounding tissue from being forced into the tissue slot. As tissue is forced into tissue slot, the surgeon will supply electrosurgical energy to the electrodes by, for example, activating the electrosurgical generator using, for example the foot switch. When the electrosurgical generator is activated, it generates an alternating electrical potential at the electrodes. The instantaneous electric potential at the third electrode is the same as the instantaneous electric potential at the fourth electrode, while the instantaneous electric potential at the first electrode is the same as the instantaneous electric potential at the second electrode. The first and second electrodes have an instantaneous electric potential which differs from and is generally of the same magnitude and opposite polarity as the electric potential applied to the third and fourth electrodes Thus, since a potential voltage is generated between electrodes of different electrical potentials, when an electrically conductive material, such as tissue, is placed in contact with both the first contact surface and the third contact surface electric current will flow between the first electrode and the third electrode. Likewise, when an electrically conductive material, such as tissue, is placed in contact with both the second contact surface and the fourth contact surface, electric current will flow between the second electrode and the fourth electrode. However, since electric current seeks the path of least resistance, which, all other variables being constant, is the shortest path, current traveling through tissue in the tissue slot will tend to travel in a first primary path between the first contact surface and the third contact surface and in a second primary path between the second contact surface and the fourth contact surface, although current will also flow in other alternative pathways.

Further, since the shortest pathway through tissue in the tissue slot is along the region adjacent the surface of the tissue, initially the current density will be highest in the region along and just below the surface of the tissue. As the tissue adjacent the top and bottom of the tissue slot begins to heat and desiccate, its electrical resistance increases and the primary current pathways are driven deeper and deeper into the tissue, toward the midpoint between the first tissue surface and the second tissue surface. This process is enhanced by the pressure on the tissue in the tissue slot which forces fluids, including water and blood, out of the tissue as it is forced into the tissue slot and which acts to facilitate coagulation and sealing of blood vessels inserted into the issue slot. The tissue slot is designed to receive the tissue to be treated while generating considerable pressure on that tissue as it is fed into the tissue slot. An end effector according to the present invention is also beneficial in that the current through the tissue tends to be self-limiting.

Once the tissue is adequately desiccated, the current flow will be reduced or eliminated, eliminating the power transfer, and thus the resistive healing in the tissue. In an end effector according to the present invention, the tissue in the tissue slot will become desiccated very quickly and the tissue reaching the tissue separator will generally be fully desiccated and any blood vessels in the tissue will be sealed on both sides of the tissue slot. Thus, as the treated tissue passes the tip of the tissue separator, it will be separated into two parts by the leading edge and guided out of the tissue slot by the first wedge wall and the second wedge wall.

In a more general sense, a bipolar electrosurgical instrument according to the present invention works by using resistive heating to cook the tissue vertically from the outer edges toward the center of the tissue and laterally working from the central portion out toward the edges of the tissue slot as the tissue moves continuously through the tissue slot As tissue near the top and bottom of the tissue slot and between insulators begins to heat, the resistance of the tissue in the treated region goes up and current is shunted to other paths with lower resistance. Thus, the current density drops off in the original volume of tissue and increases in the lower resistance regions, causing those regions to heat up. This process continues until the tissue is thoroughly treated and the resistance increases throughout the treated region. As the tissue resistance increases, the impedance load seen by the electrosurgical generator increases and the load represented by the tissue becomes mismatched with the output of the generator. With a significant load mismatch little or no additional energy is dissipated in the tissue.

An end effector according to the present invention is particularly beneficial since it means that the most critical region, that is the region in the center of tissue slot through which the leading edge passes, is the first region treated and the region that is treated most thoroughly. More particularly, by insuring that the height W_{G} of the tissue slot is less than 2 times the electrode width W_{G} the tissue in the middle of the tissue slot is cooked first and efficiently. Further, since the current in an end effector according to the present invention is self-limiting, there is little or no thermal damage to the tissue outside of the jaw assembly and the thermal damage is limited to the region necessary to ensure that the tissue on both sides of the cut line is adequately coagulated and sealed. Further, since the transfer of energy to the tissue engaged by the end effector is self regulating, the surgeon may continue to apply energy to the instrument and may move the instrument laterally through the tissue at a speed convenient to the surgeon without substantial lateral thermal damage to the tissue surrounding the jaw assembly. The surgeon may even stop the movement of the instrument through the tissue while continuing to activate the electrosurgical generator and the current to the tissue will self-limit after the tissue within the end effector is treated with little or no lateral thermal damage to the tissue outside the jaw assembly. In particular, the lateral thermal damage caused by the thermal conduction from the coagulated tissue would be expected to be typically on the order of 0.5 to 1 mm outside of the jaw assembly As an additional benefit, because the use of the instrument is not technique sensitive, for the reasons set forth above, different surgeons using the instrument in similar tissue are likely to see substantially similar results.

Bipolar electrosurgical instruments incorporating end effectors according to the present invention are particularly adapted for use in sealing and cutting individual blood vessels. Although blood vessels are generally, tough, the breakdown of collagen by electrosurgical energy and the pressure of being forced through the tissue slot will make the vessel tissue significantly weaker than normal, making it easy to mechanically ligate the treated vessel using the tissue separator. Thus, using an end effector according to the present invention, a blood vessel inserted into the tissue slot is compressed and coagulated in a single step, sealing the vessel to prevent it from leaking when it is cut. The vessel is then divided in the middle of the coagulated region, leaving two sealed ends behind, reducing or eliminating the need to use two or more mechanical clips to seal the vessel before it is cut. It may further be advantageous to use a tissue separator which includes a surgically dull leading edge since a surgically dull tissue separator will not cut healthy tissue as it is inserted into the tissue slot, thus preventing healthy tissue from being cut inadvertently as it is fed into the tissue slot prior to the application of electrosurgical energy by the surgeon. Once the tissue in the slot has been electrosurgically treated, it is desiccated and my be readily divided by the tissue separator.

It will be recognized that equivalent structures may be substituted for the structures illustrated and described herein and that the described embodiment of the invention is not the only structure which may be employed to implement the claimed invention. As one example of an equivalent structure which may be used to implement the present invention, the jaw assembly could be formed from an electrically nonconductive material with the electrodes comprising an electrically conductive material deposited on the inner surface of the tissue slot and connected by electrical conductors, such as wires, to the first electrical wire and the second electrical wire. As a further example, one or more of the electrodes may comprise a plurality of electrically conductive regions or surfaces arranged along the interior of the tissue slot to form electrode regions which cover substantially the same area on the interior surfaces of the tissue slot as the electrodes described herein. As a further example, one or more of the electrodes may include a spring mechanism adapted to force the contact surfaces against tissue positioned in the tissue slot to improve contact between tissue in the slot. A further embodiment of the present invention may only include a first electrode and a second electrode with the second tissue surface not including any electrodes and being constructed of, for example, a nonconductive material

It will be understood that the tissue effects of the present invention have not been fully characterized. It is believed that tissue in the center of the tissue slot will be fully desiccated as a result of the electrical current passing between the electrodes. Since the temperature gradient decreases in tissue away from the center of the tissue slot, tissue on either side of the center will be more likely to be coagulated and not reach total desiccation. It is further believed that steam generated in the central tissue will assist in fracturing that tissue, facilitating transaction by the tissue separator.

While preferred embodiments of the present invention have been described herein, it will be obvious to those skilled in the art that such embodiments are provided by way of example only. Numerous variations, changes, and substitutions will now occur to those skilled in the art without departing from the invention. Accordingly, it is intended that the invention be limited only by the scope of the appended claims.

## Claims

1. A bipolar electrosurgical end effector for use in medical instruments, said end effector comprising:
a first tissue surface;
a first elongated electrode on just a first side of said first tissue surface;
a second elongated electrode on just a second side of said first tissue surface, wherein said second electrode is substantially parallel to said first electrode;
a first central insulation region separating said first electrode from said second electrode;
a second tissue surface separated from said first and second electrodes by a tissue slot, said tissue slot being defined by a distance between said first tissue surface and said second tissue surface, wherein said distance is fixed; and
a tissue separator positioned between said first and second tissue surfaces, wherein said tissue separator comprises: a dividing edge at a distal end of said tissue separator, a first tissue guide extending proximally away from said dividing edge toward said first side; and
a second tissue guide extending proximally away from said dividing edge toward said second side.

2. The end effector of Claim 1 which further comprises:
a third elongated electrode on a first side of said second tissue surface;
a fourth elongated electrode on a second side of said second tissue surface, wherein said fourth elongated electrode is substantially parallel to said third elongated electrode; and
a second central insulation region separating said third electrode from said fourth electrode.

3. The end effector of Claim 2 wherein said first electrode is positioned substantially directly opposite said third electrode and said second electrode is positioned substantially directly opposite said fourth electrode.

4. The end effector of Claim 2 or Claim 3 wherein said first and second electrodes are electrically connected and said second and third electrodes are electrically connected.

5. The end effector of any one of Claims 1 to 4 wherein said height is substantially constant along the length of said tissue slot.

6. A bipolar electrosurgical end effector according to claim 1 for use in medical instruments, said end effector comprising:
the first elongated electrode on a first side of said end effector,
a third elongated electrode on said first side of said end effector, wherein said third electrode is substantially parallel to said first electrode;
the second elongated electrode on a second side of said end effector, wherein said second electrode is substantially parallel to said first and third electrodes;
a fourth elongated electrode on said second side of said end effector, wherein said fourth elongated electrode is substantially parallel to said first, second and third electrodes and wherein said first and third electrodes are separated from said first and fourth electrodes by a substantially constant gap;
the first central insulation region separating said first electrode from said second electrode;
a second central insulation region separating said third electrode from said fourth electrode; and
the tissue separator positioned between said first and said second central insulation regions proximal to the distal end of said end effector, wherein said tissue separator comprises:
a dividing edge at the distal end of said tissue separator;
a first tissue surface extending proximally away from said dividing edge toward said first side of said end effector; and
a second tissue surface extending proximally away from said dividing edge toward said second side of said end effector.

7. A bipolar electrosurgical end effector according to claim 1 for use in medical instruments, said end effector comprising:
a first inner surface extending from a proximal end of said end effector to a distal end of said end effector,
a second inner surface wherein the space between said first and second inner surfaces forms a tissue receiving gap having a fixed height at every point along said tissue slot;
the first elongated electrode on a first side of said first inner surface;
a third elongated electrode on a first side of said second inner surface wherein said third electrode is substantially parallel to said first electrode;
the second elongated electrode on a second side of said first inner surface;
a fourth elongated electrode on a second side of said second inner surface wherein said second electrode is substantially parallel to said third electrode;
the first insulation region being on said first inner surface and separating said first electrode from said second electrode;
a second insulation region on said second inner surface separating said third electrode from said fourth electrode; and
the tissue separator being a central wedge tissue separator positioned in said gap between said first and second surfaces, wherein said central wedge comprises:
a leading edge at a distal end of said central wedge;
a first tissue surface extending proximally from said leading edge toward said first side of said end effector;
a second tissue surface extending proximally from said leading edge toward said second side of said end effector.

8. The end effector according to Claim 7 wherein said first inner surface is substantially parallel to said second inner surface.

9. The end effector of any one of Claims 6 to 8 wherein said first electrode is positioned substantially directly opposite said second electrode and said third electrode is positioned substantially directly opposite said fourth electrode.

10. The end effector of any one of claims 6 to 9 wherein said first and third electrodes are electrically connected and said second and fourth electrodes are electrically connected.

11. The end effector of any one of claims 1 to 10 wherein said tissue separator further comprises a wedge shaped region.

12. The end effector of any one of claims 1 to 11 wherein said dividing edge is surgically dull.

## Patentansprüche

1. Bipolarer elektrochirurgischer Effektor zur Anwendung in medizinischen Instrumenten, wobei genannter Effektor umfaßt:
eine erste Gewebefläche;
eine erste längliche Elektrode auf genau einer ersten Seite von genannter erster Gewebefläche;
eine zweite längliche Elektrode auf genau einer zweiten Seite von genannter erster Gewebefläche, wobei genannte zweite Elektrode im wesentlichen parallel zu genannter erster Elektrode ist;
ein erstes zentrales Isoliergebiet, das genannte erste Elektrode von genannter zweiter Elektrode trennt;
eine zweite Gewebefläche, die von genannten ersten und zweiten Elektroden durch einen Gewebeschlitz getrennt ist, wobei genannter Gewebeschlitz durch einen Abstand zwischen genannter erster Gewebefläche und genannter zweiter Gewebefläche definiert ist, wobei genannter Abstand fest ist; und
eine Gewebetrenneinrichtung, die zwischen genannten ersten und zweiten Gewebeflächen positioniert ist, wobei genannte Gewebetrenneinrichtung umfaßt:
eine Teilkante an einem distalen Ende von genannter Gewebetrenneinrichtung, eine erste Gewebeführung, die sich von genannter Teilkante in Richtung auf genannte erste Seite proximal weg erstreckt, und eine zweite Gewebeführung, die sich von genannter Teilkante in Richtung auf genannte zweite Seite proximal weg erstreckt.

2. Effektor nach Anspruch 1, **dadurch gekennzeichnet, daß** er ferner umfaßt:
eine dritte längliche Elektrode auf einer ersten Seite von genannter zweiter Gewebefläche;
eine vierte längliche Elektrode auf einer zweiten Seite von genannter zweiter Gewebefläche, wobei genannte vierte längliche Elektrode im wesentlichen parallel zu genannter dritter länglicher Elektrode verläuft, und
ein zweites zentrales Isoliergebiet, das genannte dritte Elektrode von genannter vierter Elektrode trennt.

3. Effektor nach Anspruch 2, **dadurch gekennzeichnet, daß** genannte erste Elektrode im wesentlichen direkt gegenüber genannter dritter Elektrode positioniert ist und genannte zweite Elektrode im wesentlichen direkt gegenüber von genannter vierter Elektrode positioniert ist.

4. Effektor nach Anspruch 2 oder Anspruch 3, **dadurch gekennzeichnet, daß** genannte erste und zweite Elektroden elektrisch verbunden sind und genannte zweite und dritte Elektroden elektrisch verbunden sind.

5. Effektor nach einem der Ansprüche 1 bis 4, **dadurch gekennzeichnet, daß** genannte Höhe entlang der Länge von genanntem Gewebeschlitz im wesentlichen konstant ist.

6. Bipolarer elektrochirurgischer Effektor nach Anspruch 1 zur Anwendung in medizinischen Instrumenten, wobei genannter Effektor umfaßt:
die erste längliche Elektrode auf einer ersten Seite von genanntem Effektor;
eine dritte längliche Elektrode auf genannter erster Seite von genanntem Effektor, wobei genannte dritte Elektrode im wesentlichen parallel zu genannter erster Elektrode verläuft;
die zweite längliche Elektrode auf einer zweiten Seite von genanntem Effektor, wobei genannte zweite Elektrode im wesentlichen parallel zu genannten ersten und dritten Elektroden verläuft;
eine vierte längliche Elektrode auf genannter zweiter Seite von genanntem Effektor, wobei genannte vierte längliche Elektrode im wesentlichen parallel zu genannten ersten, zweiten und dritten Elektroden verläuft und wobei genannte erste und dritte Elektroden von genannten dritten und vierten Elektroden durch einen im wesentlichen konstanten Spalt getrennt sind;
das erste zentrale Isoliergebiet, das genannte erste Elektrode von genannter zweiter Elektrode trennt;
ein zweites zentrales Isoliergebiet, das genannte dritte Elektrode von genannter vierter Elektrode trennt; und
die Gewebetrenneinrichtung, die zwischen genannten ersten und zweiten zentralen Isoliergebieten proximal zum distalen Ende von genanntem Effektor positioniert ist, wobei genannte Gewebetrenneinrichtung umfaßt:
eine Teilkante am distalen Ende von genannter Gewebetrenneinrichtung;
eine erste Gewebefläche, die sich von genannter Teilkante in Richtung auf genannte erste Seite von genanntem Effektor proximal weg erstreckt; und
eine zweite Gewebefläche, die sich von genannter Teilkante in Richtung auf genannte zweite Seite von genanntem Effektor proximal weg erstreckt.

7. Bipolarer elektrochirurgischer Effektor nach Anspruch 1 zur Anwendung in medizinischen Instrumenten, wobei genannter Effektor umfaßt:
einer erste Innenfläche, die sich von einem proximalen Ende von genanntem Effektor zu einem distalen Ende von genanntem Effektor erstreckt;
eine zweite Innenfläche, wobei der Raum zwischen genannten ersten und zweiten Innenflächen einen Gewebeaufnahmespalt mit einer festen Höhe an jedem Punkt entlang von genanntem Gewebeschlitz bildet;
die erste längliche Elektrode auf einer ersten Seite von genannter erster Innenfläche;
eine dritte längliche Elektrode auf einer ersten Seite von genannter zweiter Innenfläche, wobei genannte dritte Elektrode im wesentlichen parallel zu genannter erster Elektrode verläuft;
die zweite längliche Elektrode auf einer zweiten Seite von genannter erster Innenfläche;
eine vierte längliche Elektrode auf einer zweiten Seite von genannter zweiter Innenfläche, wobei genannte zweite Elektrode im wesentlichen parallel zu genannter dritter Elektrode verläuft;
wobei sich das erste Isoliergebiet auf genannter erster Innenfläche befindet und genannte erste Elektrode von genannter zweiter Elektrode trennt;
ein zweites Isoliergebiet auf genannter zweiter Innenfläche, das genannte dritte Elektrode von genannter vierter Elektrode trennt; und
die Gewebetrenneinrichtung eine Gewebetrenneinrichtung mit einem zentralen Keil ist, die in genanntem Spalt zwischen genannten ersten und zweiten Flächen positioniert ist, wobei genannter zentraler Keil umfaßt:
eine Vorderkante an einem distalen Ende von genanntem zentralen Keil;
eine erste Gewebefläche, die sich von genannter Vorderkante in Richtung auf genannte erste Seite von genanntem Effektor proximal erstreckt;
eine zweite Gewebefläche, die sich von genannter Vorderkante in Richtung auf genannte zweite Seite von genanntem Effektor proximal erstreckt.

8. Effektor nach Anspruch 7, **dadurch gekennzeichnet, daß** genannte Innenfläche im wesentlichen parallel zu genannter zweiter Innenfläche verläuft.

9. Effektor nach einem der Ansprüche 6 bis 8, **dadurch gekennzeichnet, daß** genannte erste Elektrode im wesentlichen direkt gegenüber genannter zweiter Elektrode positioniert ist und genannte dritte Elektrode im wesentlichen direkt gegenüber genannter vierter Elektrode positioniert ist.

10. Effektor nach einem der Ansprüche 6 bis 9, **dadurch gekennzeichnet, daß** genannte erste und dritte Elektroden elektrisch verbunden sind und genannte zweite und vierte Elektroden elektrisch verbunden sind.

11. Effektor nach einem der Ansprüche 1 bis 10, **dadurch gekennzeichnet, daß** genannte Gewebetrenneinrichtung ferner ein keilförmiges Gebiet umfaßt.

12. Effektor nach einem der Ansprüche 1 bis 11, **dadurch gekennzeichnet, daß** genannte Teilkante chirurgisch stumpf ist.

## Revendications

1. Effecteur terminal électrochirurgical bipolaire pour utilisation dans des instruments médicaux, ledit effecteur terminal comprenant :
une première surface tissulaire ;
une première électrode allongée sur juste un premier côté de ladite première surface tissulaire ;
une deuxième électrode allongée sur juste un second côté de ladite première surface tissulaire, dans laquelle ladite deuxième électrode est sensiblement parallèle à ladite première électrode ;
une première région d'isolation centrale séparant ladite première électrode de ladite deuxième électrode ;
une seconde surface tissulaire séparée desdites première et deuxième électrodes par une fente de tissu, ladite fente de tissu étant définie par une distance entre ladite première surface tissulaire et ladite seconde surface tissulaire, dans laquelle ladite distance est fixe ; et
un séparateur de tissu positionné entre lesdites première et seconde surfaces tissulaires, dans lesquelles ledit séparateur de tissu comprend : un bord de division à une extrémité distale dudit séparateur de tissu ; un premier guide de tissu s'étendant à une distance proximale dudit bord de division vers ledit premier côté ; et un second guide de tissu s'étendant à une distance proximale dudit bord de division vers ledit second côté.

2. Effecteur terminal selon la revendication 1 qui comprend en outre :
une troisième électrode allongée sur un premier côté de ladite seconde surface tissulaire ;
une quatrième électrode allongée sur un second côté de ladite seconde surface tissulaire, dans laquelle ladite quatrième électrode allongée est sensiblement parallèle à ladite troisième électrode allongée ; et
une seconde région d'isolation centrale séparant ladite troisième électrode de ladite quatrième électrode.

3. Effecteur terminal selon la revendication 2, dans lequel ladite première électrode est positionnée sensiblement directement à l'opposé de ladite troisième électrode et ladite deuxième électrode est positionnée sensiblement directement à l'opposé de ladite quatrième électrode.

4. Effecteur terminal selon la revendication 2 ou la revendication 3, dans lequel lesdites première et deuxième électrodes sont électriquement connectées et lesdites deuxième et troisième électrodes sont électriquement connectées.

5. Effecteur terminal selon une quelconque des revendications 1 à 4, dans lequel ladite hauteur est sensiblement constante le long de la longueur de ladite fente de tissu.

6. Effecteur terminal électrochirurgical bipolaire selon la revendication 1 pour utilisation dans des instruments médicaux, ledit effecteur terminal comprenant :
la première électrode allongée sur un premier côté dudit effecteur terminal ;
une troisième électrode allongée sur ledit premier côté dudit effecteur terminal, dans lequel ladite troisième électrode est sensiblement parallèle à ladite première électrode ;
la deuxième électrode allongée sur un deuxième côté dudit effecteur terminal, dans lequel ladite deuxième électrode est sensiblement parallèle aux dites première et troisième électrodes ;
une quatrième électrode allongée sur ledit second côté dudit effecteur terminal, dans lequel ladite quatrième électrode allongée est sensiblement parallèle aux dites première, deuxième et troisième électrodes et dans lequel lesdites première et troisième électrodes sont séparées desdites troisième et quatrième électrodes par un vide sensiblement constant ;
la première région d'isolation centrale séparant ladite première électrode de ladite deuxième électrode ;
une seconde région d'isolation centrale séparant ladite troisième électrode de ladite quatrième électrode ; et
le séparateur de tissu positionné entre ladite première et ladite seconde régions d'isolation centrale proximalement à l'extrémité distale dudit effecteur terminal, dans lequel ledit séparateur de tissu comprend :
un bord de division à l'extrémité distale dudit séparateur de tissu ;
une première surface tissulaire s'étendant à une distance proximale dudit bord de division vers ledit premier côté dudit effecteur terminal ; et
une seconde surface tissulaire s'étendant à une distance proximale dudit bord de division vers ledit second côté dudit effecteur terminal.

7. Effecteur terminal électrochirurgical bipolaire selon la revendication 1 utilisé dans des instruments médicaux, ledit effecteur terminal comprenant :
une première surface interne s'étendant depuis une extrémité proximale dudit effecteur terminal vers une extrémité distale dudit effecteur terminal ;
une seconde surface interne dans laquelle l'espace entre lesdites première et seconde surfaces internes forme un vide recevant le tissu ayant une hauteur fixe à tout point le long de ladite fente de tissu ;
la première électrode allongée sur un premier côté de ladite première surface interne ;
une troisième électrode allongée sur un premier côté de ladite seconde surface interne dans laquelle ladite troisième électrode est sensiblement parallèle à ladite première électrode ;
la deuxième électrode allongée sur un second côté de ladite première surface interne ;
une quatrième électrode allongée sur un second côté de ladite seconde surface interne dans laquelle ladite deuxième électrode est sensiblement parallèle à ladite troisième électrode ;
la première région d'isolation étant sur ladite première surface interne et séparant ladite première électrode de ladite deuxième électrode ;
une seconde région d'isolation sur ladite seconde surface interne séparant ladite troisième électrode de ladite quatrième électrode ; et
le séparateur de tissu étant un séparateur de tissu à cale centrale positionné dans ledit vide entre lesdites première et seconde surfaces, dans lequel ladite cale centrale comprend :
un bord d'attaque à une extrémité distale de ladite cale centrale ;
une première surface tissulaire s'étendant à une distance proximale dudit bord d'attaque vers ledit premier côté dudit effecteur terminal ;
une seconde surface tissulaire s'étendant à une distance proximale dudit bord d'attaque vers ledit second côté dudit effecteur terminal.

8. Effecteur terminal selon la revendication 7, dans lequel ladite première surface interne est sensiblement parallèle à ladite seconde surface interne.

9. Effecteur terminal selon une quelconque des revendications 6 à 8, dans lequel ladite première électrode est positionnée sensiblement directement à l'opposé de ladite deuxième électrode, et ladite troisième électrode est positionnée sensiblement directement à l'opposé de ladite quatrième électrode.

10. Effecteur terminal selon une quelconque des revendications 6 à 9, dans lequel lesdites première et troisième électrodes sont électriquement connectées et lesdites deuxième et quatrième électrodes sont électriquement connectées.

11. Effecteur terminal selon une quelconque des revendications 1 à 10, dans lequel ledit séparateur de tissu comprend en outre une région en forme de cale.

12. Effecteur selon une quelconque des revendications 1 à 11, dans lequel ledit bord de division est chirurgicalement mat.
